(19) Europäisches Patentamt
European Patent Office
Office européen des brevets

(11) Veröffentlichungsnummer : **0 075 751**
**B1**

(12) # EUROPÄISCHE PATENTSCHRIFT

(45) Veröffentlichungstag der Patentschrift : **11.12.85**

(51) Int. Cl.⁴ : **C 11 D 3/39**, C 07 D249/12

(21) Anmeldenummer : **82108183.3**

(22) Anmeldetag : **06.09.82**

(54) **Triazolidin-3,5-dione als Aktivatoren für Perverbindungen.**

(30) Priorität : **16.09.81 DE 3136808**

(43) Veröffentlichungstag der Anmeldung : **06.04.83 Patentblatt 83/14**

(45) Bekanntmachung des Hinweises auf die Patenterteilung : **11.12.85 Patentblatt 85/50**

(84) Benannte Vertragsstaaten : **CH DE FR GB LI**

(56) Entgegenhaltungen :
**EP-A- 0 044 417**
**EP-A- 0 044 421**
**DE-A- 1 961 076**
**DE-A- 2 126 538**
**DE-A- 2 947 619**
**DE-A- 3 012 922**
**Dictionary of Organic Compounds (Chapman and Hall)**
**Beilsteins Handbuch der Organischen Chemie**

(73) Patentinhaber : **BAYER AG**
**Konzernverwaltung RP Patentabteilung**
**D-5090 Leverkusen 1 Bayerwerk (DE)**

(72) Erfinder : **Schellhammer, Karl-Wolfgang, Dr.**
**Katharinental 26**
**D-5060 Bergisch-Gladbach 2 (DE)**
Erfinder : **Rottmaier, Ludwig**
**Bergstrasse 85**
**D-5068 Odenthal-Glöbusch (DE)**
Erfinder : **Merten, Rudolf, Dr.**
**Berta-von-Suttner-Strasse 55**
**D-5090 Leverkusen 1 (DE)**
Erfinder : **Schimmel, Ulrich**
**Richard-Wagner-Strasse 13**
**D-5090 Leverkusen 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 075 751 B1

**Beschreibung**

Die Erfindung betrifft die Verwendung von acylierten Triazolidin-3,5-dionen als Aktivatoren für Perverbindungen, Bleich- und Waschmittel, enthaltend diese Aktivatoren.

Es ist bekannt, daß anorganische Perverbindungen, insbesondere Perborate und Percarbonate, als Wirksubstanzen Bestandteil zahlreicher Bleichmittel, wie sie z. B. zum Bleichen und/oder gleichzeitigem Waschen von Textilien verwendet werden, sind. Die Wirkung derartiger Bleichmittel ist jedoch erst im Temperaturbereich zwischen 70 °C und 100 °C optimal. Da jedoch bei dieser Temperatur der Energieaufwand sehr hoch ist und da viele Textilien aus synthetischen Fasern bzw. aus Mischungen von synthetischen und natürlichen Fasern bestehen und somit gegen höhere Waschtemperaturen sehr empfindlich sind, wird angestrebt, die Waschtemperatur auf 60 °C und niedriger, daß heißt bis 30 °C, zu senken.

Bei den Versuchen, die Bleich- und/oder Waschtemperatur zu senken, hat man gefunden, daß Perverbindungen durch Zusatz von sogenannten Aktivatoren, insbesondere von abspaltbaren N-Acyl- und O-Acylverbindungen, auch bei neidrigeren Temperaturen, d. h. unterhalb 60 °C, wirksam werden. So wird z. B. in der DE-A-1 961 775 die Verwendung von Tetraacetylethylendamin, in der DE-A-2 126 538 u. a. die Verwendung von Tetraacetylmethylendiamin, Trisacetylcyanurat, Acetylsalicylsäure, in der DE-A-1 961 076 die Verwendung von acylierten Glykolurilen, in der DE-A-2 051 554 die Verwendung von acylierten 2,5-Diketopiperazinen beschrieben.

Es wird nun eine weitere Gruppe von Aktivatoren zur Verfügung gestellt, welche leicht zugänglich sind und welche in der Wirksamkeit den bisher bekannten Aktivatoren überlegen sind.

Die vorliegende Erfindung betrifft nun die Verwendung von Verbindungen der Formel

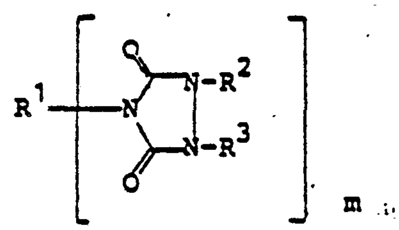

(I)

in der

$R^1$ Wasserstoff oder einen organischen Rest bedeutet, wobei die Bindung $R^1$-N eine C-N-Bindung ist ;

$R^2$, $R^3$ für Wasserstoff oder einen organischen Rest stehen, wobei die Bindungen $R^2$-N und $R^3$-N C-N-Bindungen sind, und

m 1, 2, 3, 4 oder 5 bedeutet,

mit der Maßgabe, daß mindestens einer der Reste $R^1$, $R^2$ oder $R^3$ für eine Acylgruppe steht, als Aktivatoren für Perverbindungen.

Die C-Atome des organischen Restes $R^1$, an welche die N-Atome der Triazolidin-3,5-dion-Reste gebunden sind, gehören vorzugsweise aliphatischen, aromatischen oder gemischt aliphatisch-aromatischen Systemen an.

Erfindungsgemäß ist der organische Rest $R^1$ durch 1 bis 5, bevorzugt 1 bis 3, Triazolidin-3,5-dion-Reste substituiert.

Vorzugsweise handelt es sich um Reste von $C_1$-$C_{20}$-Alkanen, $C_1$-$C_{12}$-Alkanen, $C_1$-$C_6$-Alkanen, $C_3$-$C_7$-Cycloalkanen, Benzol, Naphthalin, Phenyl-$C_1$-$C_6$-alkanen, wobei die genannten Kohlenwasserstoffe substituiert sein können oder es handelt sich um Reste von Kohlenwasserstoffen des Typs

$$X_1—(C_nH_{2n}—X_2)_n—X_3$$

wobei $X_1$, $X_2$, $X_3$ gegebenenfalls substituiertes Cyclopentyl, gegebenenfalls substituiertes Cyclohexyl oder gegebenfalls substituiertes Phenyl bezeichnen, wobei $X_1$, $X_2$ und $X_3$ vorzugsweise die gleiche Bedeutung haben und n 1, 2, 3 oder 4 bezeichnet oder es handelt sich um Reste von aliphatischen Ethern mit 2-20 C-Atomen, aromatischen Ethern mit 12-24 C-Atomen, gemischt aliphatischaromatischen Ethern mit 7-24 C-Atomen, aliphatisch tertiären Aminen mit 3-20 C-Atomen oder aromatischen tertiären Aminen mit 8-24 C-Atomen.

Die für $R^1$ genannten Kohlenwasserstoffreste können z. B. durch ($C_1$-$C_4$-Alkoxy)-carbonyl, CN, Halogen wie Cl, Br und F und im Falle aromatischer Reste auch durch Niedrigalkyl, insbesondere $C_1$-$C_4$-Alkyl, substituiert sein.

Formelmäßig seien beispielhaft folgende bevorzugte Reste $R^1$ angegeben :

a) $C_nH_{2n+1}—$ $\quad n = 1$ bis 8

b) $—(CH_2)_m—$ $\quad m = 2$ bis 12

c) 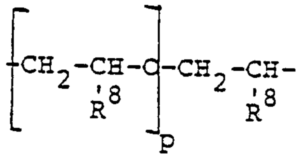

$R^8$ = H, $CH_3$
p = 1-9

d) 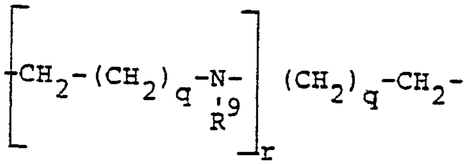

$R^9$ $C_1$-$C_4$-Alkyl
q = 1-2
r = 1-4

e) 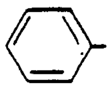 , 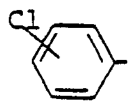

f) 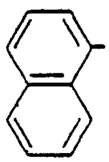

g) 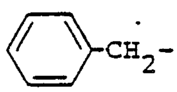

h) 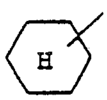

i) 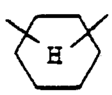

j) 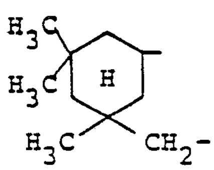

k) 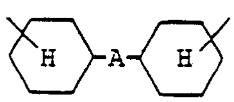

A = Alkylen mit 1-4 C-Atomen,
O, —N($CH_3$)—,

l) 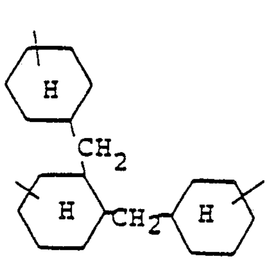

m) 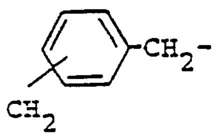

n) 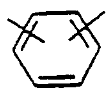

o) 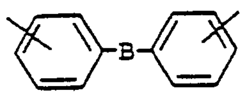 B = Alkylen mit 1-4 C-Atomen, O, $-N(CH_3)-$,

p) 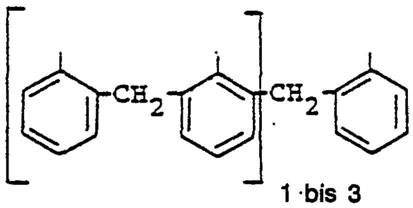
1 bis 3

q) $C_4H_9-O-CH_2-CH_2-CH_2-$

r) $-(CH_2)_3-O-(CH_2)_{2\ bis\ 4}-O-(CH_2)_3-$

s) 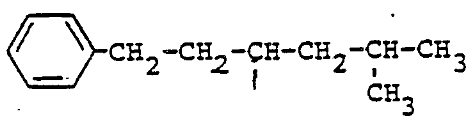

t) H–

u) $C_nH_{2n+1}-\overset{O}{\overset{\|}{C}}-$ n = 1 bis 3

v) 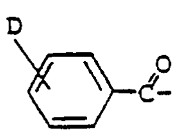 D = H, Cl, $CH_3$, $NO_2$.

$R^2$, $R^3$ bezeichnen bevorzugt Wasserstoff, $C_1$-$C_{10}$-Alkyl, das z. B. durch Halogen wie Chlor, Brom, Fluor oder durch Cyan substituiert sein kann und besonders bevorzugt Acylreste, wie ($C_1$-$C_8$-Alkyl)-carbonyl, Benzoyl oder Phenyl-($C_1$-$C_4$-alkyl)-carbonyl, wobei die genannten Acylreste weitere Substituenten wie beispeilsweise $C_1$-$C_4$-Alkoxy, Halogen wie Cl, Br, F, Nitro, Cyan tragen können.

Beispiele für $R^1$, $R^2$ sind : Acetyl, Propionyl, n-Butyryl, i-Butyryl, Benzoyl, Toluoyl, Xyloyl, m-Chlorbenzoyl, m-Nitrobenzoyl, p-Nitrobenzoyl.

In Formel I können $R^2$ und $R^3$ verschieden sein ; jedoch werden insbesondere Verbindungen mit gleichen Acylresten bevorzugt.

Bevorzugt sind Verbindungen der Formel I mit

m = 1 oder 2 und $R^2 = R^3$ = Acetyl, Propionyl oder Benzoyl sowie Verbindungen mit

m = 1 und $R^1 = R^2 = R^3$ = Acetyl, Propionyl oder Benzoyl.

m in Formel I steht bevorzugt für 1, 2 oder 3.

Erfindungsgemäß besonders bevorzugt werden 1,2,4-Triacetyl-triazolidin-3,5-dion, 1,2-Bisacetyl-triazolidin-3,5-dion und 1,2-Ethan-diyl-4,4'-bis-[1,2-bis-acetyltriazolidin-3,5-dion].

Zur Herstellung von Verbindungen der Formel I acyliert man Verbindungen der Formel II

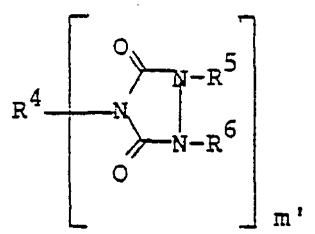 (II)

in der

$R^4$ Wasserstoff, einen organischen Rest, wobei die Bindungen $R^4$-N C-N-Bindungen sind, und für den Fall, daß m' = 1 ist auch Acyl,

$R^5$, $R^6$ Wasserstoff, einen organischen Rest, wobei die Bindungen $R^5$-N und $R^6$-N C-N-Bindungen sind, Acyl und

m' 1, 2, 3, 4 oder 5 bezeichnen,

wobei mindestens einer der Reste $R^4$, $R^5$, $R^6$ für Wasserstoff steht.

Die Verbindungen der Formel II können nach literaturbekannten Verfahren hergestellt werden.

1,2,4-Triazolidin-3,5-dion kann aus Hydrazodicarbonamid, suspendiert in einem organischen, gegebenenfalls mit Wasser mischbaren Lösungsmittel, z. B. N-Methyl-pyrrolidon, durch Cyclisierung bei Temperaturen von 150 °C bis 280 °C und einem Druck von 50 mbar bis 5 bar unter Abspaltung von Ammoniak und Entfernung des abgespaltenen Ammoniaks aus dem Reaktionsgemisch hergestellt und nach Kristallisation isoliert werden (vgl. DE-A-2 947 619).

Ausgangsverbindungen der Formel II, in der m' 1-5, $R^5$ und $R^6$ Wasserstoff und $R^4$ einen einwertigen Rest bzw. m = 2 bis 5, $R^5$ und $R^6$ Wasserstoff und $R^4$ einen zweibis fünfwertigen Rest darstellen, können nach den vorstehend beschriebenen Verfahrensschritten aus Hydrazodicarbonamid und einem primären Monoamin bzw. einem Polyamin mit 2 bis 5 primären Aminogruppen unter Abspaltung von Ammoniak erhalten werden, wobei auf eine primäre Aminogruppe vorzugsweise 0,9 bis 1,1 Mol Hydrazodicarbonamid eingesetzt werden.

Diese Ausgangsverbindungen der allgemeinen Formel II können, wie z. B. im chemischen Zentralblatt *1898* I, 39 beschrieben, durch Acylierung mit dem entsprechenden Anhydrid der Formel

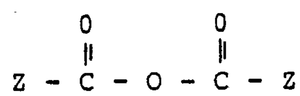

in der Z $C_1$ bis $C_8$-Alkyl, Phenyl oder Phenyl ($C_1$ bis $C_4$)-alkyl bedeutet, zu den erfindungsgemäßen Aktivatoren der allgemeinen Formel I umgesetzt werden. So kann das 1,2-Bis-acetyltriazolidin-3,5-dion aus 1,2,4-Triazolidin-3,5-dion durch 1/2-stündiges Kochen mit Acetanhydrid erhalten werden. Die nicht literaturbekannten acylierten Triazolidin-3,5-dione der allgemeinen Formel I können analog hergestellt werden.

Die erfindungsgemäßen Aktivatoren für Perverbindungen, insbesondere anorganische Perverbindungen, der allgemeinen Formel I zeichnen sich durch eine sehr gute aktivierende Wirkung und eine gute Löslichkeit in Wasser aus. Die mit den acylierten Triazolidin-3,5-dionen als Aktivatoren hergestellten Bleich- und/oder bleichenden Waschmittel zeigen in den meisten Fällen eine deutlich bessere Aktivierung als die derzeit bekannten Aktivatoren.

Bei der Auswahl der den Bleich- oder Waschmitteln zuzugebenden Mengen an Aktivierungsmittel kann im allgemeinen davon ausgegangen werden, daß jede abspaltbare Acylgruppe ein Aktivsauerstoffatom der verwendeten anorganischen Perverbindung zu aktivieren vermag. Für eine vollständige Aktivierung des eingesetzten Aktivsauerstoffs der anorganischen Perverbindungen sind daher theoretisch äquivalente Mengen an Aktivierungsmittel und organischer Perverbindung anzuwenden. In der Praxis ist jedoch häufig eine wesentlich niedrigere Menge an Aktivator ausreichend. Andererseits ist es auch möglich, den Aktivator in einem großen Überschuß gegenüber der Perverbindung einzusetzen. Im allgemeinen wird die einzusetzende Menge an Aktivator in einem Verhältnis von 0,1 bis 6, vorzugsweise 0,2 bis 1 abspaltbarer Acylreste pro Aktivsauerstoffatom der Perverbindung liegen. In der Praxis wird das angewandte Verhältnis von Aktivator zu anorganischer Perverbindung meistens etwa 1 : 2 bis 1 : 3 betragen.

Ein besonderer Vorteil bei der Verwendung der Verbindungen der allgemeinen Formel I in Perverbindungen enthaltenden Bleich- und/oder bleichenden Waschmittel ist, daß bereits bei niedrigeren Temperaturen, besonders bei Temperaturen von 40 bis 60 °C eine Aktivierung der Perverbindungen und eine Beschleunigung des Bleich- und Waschvorgangs eintritt. Diese Beschleunigung des Bleich- und Waschvorgangs ist auch bei höheren Temperaturen bis zu 100 °C noch zu beobachten. Diese Aktivierung und Beschleunigung des Bleich- und Waschvorganges erlaubt es, bei gleichbleibender Wirkung die Behandlungstemperatur zu senken und/oder die Waschdauer zu verkürzen, was zu erheblichen Zeit- und/oder Energieeinsparungen beim Waschvorgang führt.

Die in der Praxis bei dem jeweiligen Bleich- und Waschproblem anzuwendenden Bedingungen wie Temperatur und Dauer der Behandlung, Konzentration der Perverbindung und des Aktivators sowie der pH-Wert der Waschflotte, richten sich nach den zu bleichenden oder waschenden Waren oder nach dem beim Bleichprozeß vorhandenen Begleitmaterial. Die Konzentration der Perverbindungen in den meist wäßrigen Bleich- oder bleichenden Waschflotten ist vor allem abhängig vom angestrebten Bleicheffekt und wird im allgemeinen so eingestellt, daß die Waschflotten etwa 10 bis 500, vorzugsweise 50 bis 300 mg Aktivsauerstoff/Liter enthalten.

Der Bleich- und/oder Waschvorgang wird normalerweise bei einem pH-Wert von 6-12, vorzugsweise 8-11, durchgeführt. Da die Wirkung der Aktivatoren jedoch mit einem Alkaliverbrauch verbunden ist, sollten in den bekannten Waschmittel-Zusammensetzungen ausreichende Mengen an stark alkalisch reagierenden Salzen oder entsprechende Puffersubstanzen enthalten sein, um ein Absinken des pH-Wertes der Bleich- bzw. Waschflotte unter den gewüschten Wert zu verhindern. Geeignete alkalisch reagierende Salze oder Puffersubstanzen, welche den pH-Wert zwischen 6 und 12 halten, sind zum Beispiel Phosphate, Carbonate, Bicarbonate oder Silikate der Alkalimetalle. Beispielhaft seien genannt : Natriumbicarbonat, Natriumcarbonat, Kaliumcarbonat, Natriumsilikat, Dinatriumhydrogenphosphat, Natriumdihydrogenphosphat.

Die erfindungsgemäßen Aktivatoren finden in Kombination mit anorganischen Perverbindungen bevorzugt Anwendung in Bleich- und/oder bleichenden Waschmitteln für Textilien. Als Textilien kommen neben den normalerweise bei höheren Temperaturen von 80-100 °C zu behandelnden Waren aus Leinen und Baumwolle, vor allem auch die aus Regeneratcellulose und anderen synthetischen Fasern sowie aus deren Mischungen hergestellten oder diese enthaltenden Waren in Betracht, welche bei niederen Temperaturen, vorzugsweise bei 40 bis 60 °C, gebleicht oder gewaschen werden. Ein besonderer Vorteil der erfindungsgemäßen Aktivatoren für anorganische Perverbindungen ist, daß bei der Bleiche oder Wäsche bereits bei diesen niederen Temperaturen eine ausreichende Aktivierung der Perverbindung erfolgt und ein guter Bleicheffekt erzielt wird.

Als Perverbindungen werden vorzugsweise anorganische Perverbindungen, welche in wäßriger Lösung $H_2O_2$ liefern, verwendet. Dabei kommen vorwiegend die Perborate, Persilikate, Percarbonate und Peroxyhydrate der Ortho-, Pyro- oder Polyphosphate der Alkalimetalle, insbesondere die Natrium- und Kaliumsalze in Betracht. Als besonders bevorzugte Perverbindung wird das Natriumperborattetrahydrat verwendet.

Die erfindungsgemäßen Aktivatoren können als Pulver oder Granulate leicht mit den übrigen Bestandteilen der Bleich-, Wasch- oder Waschhilfsmittel vermischt werden. Im allgemeneinen werden die besonders bevorzugten bleichenden Waschmittel erhalten, indem man ein durch Zerstäubungstrocknung gewonnenes Waschmittelpulver, welches noch keine Perverbindung enthält, mit den pulvrigen oder körnigen Aktivatoren sowie mit der Perverbindung vermischt. Enthalten die erfindungsgemäßen Waschmittel größere Mengen nichttensidische Schauminhibitoren und/oder flüssig bzw. pastös vorliegende nichtionische Tenside, so ist es möglich, diese nach dem Sprühnebelmischverfahren auf das Waschmittelpulver aufzusprühen und das erhaltene Produkt mit den übrigen Bestandteilen zu mischen.

Die erfindungsgemäßen bleichenden Waschmittel können auch in zweigeteilter Form vorliegen, wobei der eine Teil den erfindungsgemäßen Aktivator und der andere die Waschmittelkomponente, die Perverbindung und gegebenenfalls weitere Zusätze enthält. Der Aktivator kann dabei in anwendungsgerechten Teilmengen fauchtigkeitsgeschützt abgepackt vorliegen und z. B. in Form von Tabletten oder als Portionspackungen zur gemeinsamen Verwendung mit aktivatorfreien bleichenden Waschmitteln verwendet werden. Bei der praktischen Anwendung wird der getrennt verpackt vorliegende Aktivator kurz vor oder während des Waschvorgangs der Waschflotte zugegeben.

Zur Verbesserung der Lagerbeständigkeit der erfindungsgemäßen Aktivatoren können diese in einer solchen Beschaffenheit vorliegen, daß sie mit den übrigen Bestandteilen der Bleich- oder bleichenden Waschmittel, insbesondere mit den erfindungsgemäß mitzuverwendenden Perverbindungen, nicht in direkten Kontakt kommen und somit auch nicht vorzeitig in Reaktion treten können. Obwohl sich die acylierten Triazolidin-3,5-dione der allgemeinen Formel I durch eine gute Hydrolysebeständigkeit auszeichnen, ist es vorteilhaft, diese gegebenenfalls mit einem Metallsalz und/oder mit einem Komplexbildner, zu granulieren und durch Umhüllung vor Feuchtigkeit sowie vor Kontakt mit der Perverbindung zu schützen. Als Hüllmaterialien sind wasserlösliche oder wasserunlösliche, insbesondere in Wasser quellbare organische Substanzen wie z. B. Gelatine, Methyl-, Ethyl-, Oxyethyl- und Carboxymethylcellulose sowie Polyglykolether, langkettige Fettsäuren und Alkohole oder Polyvinylalkohole geeignet.

Dieses Umhüllen kann nach bekannten Verfahren, wie es z. B. durch Umhüllen mit Fettsäuren (DE-A-2 221 492), mit Gemischen aus Fettsäuren und Polyglykolen (DE-A-2 207 974) und mit Gemischen aus Polyvinylalkohol und Carboxymethylzellulose (GB-A-907 358) bei bekannten N-Acylverbindungen beschrieben wird, erfolgen.

Die unter Verwendung der erfindungsgemäßen Aktivatoren hergestellten Bleich- oder bleichenden Waschmitel können ferner die für diese Mittel üblichen Zusammensetzungen aufweisen. Dabei kann die Mischung aus Aktivator und Perverbindung 10 bis 100 Gew.-% der Gesamtzusammensetzung ausmachen. Bei Textilwaschmitteln beträgt der Anteil an Perverbindungen und Aktivatoren etwa 5 bis 50, vorzugsweise 5 bis 30 Gew.-%. Die übrigen Komponenten solcher Waschmittel sind vor allem Tenside mit einem

Anteil von etwa 5 bis 40, vorzugsweise 10 bis 30 Gew.-%, Gerüststoffe, sogenannte Builder, mit einem Anteil von etwa 10 bis 80, vorzugsweise 30 bis 75 Gew.-% und sonstige Waschmittelbestandteile und Hilfs- oder Zusatzstoffe wie z. B. Schmutzträger, optische Aufheller, Farbstoffe, Duftstoffe, Enzyme, Schauminhibitoren, hydrotrope Substanzen und Wasser mit einem Anteil von etwa 0 bis 15, vorzugsweise 1 bis 10 Gew-%.

Als waschaktive Substanzen werden die dafür bekannten Verbindungen, welche anionischen, kationischen, zwitterionischen oder nichtionischen Charakter haben können, verwendet. Diese Verbindungen enthalten wenigstens einen hydrophoben Rest von meist 8-26, vorzugsweise 12-18 C-Atomen und wenigstens eine anionische, kationische, zwitterionische oder nichtionische wasserlöslichmachende Gruppe. Der hydrophobe Rest ist meistens ein gesättigter aliphatischer oder alicyclischer Rest, vorzugsweise ein aliphatischer Rest mit vorzugsweise 12-18 C-Atomen.

Er kann mit den wasserlöslichmachenden Gruppen direkt oder über Zwischenglieder z. B. über Benzolringe, Carbonsäureester- oder Carbonamidgruppen, ether- oder esterartig gebundene Reste mehrwertiger Alkohole oder Polyetherreste, verknüpft sein.

Als anionische Tenside sind Seifen aus natürlichen oder synthetischen Fettsäuren brauchbar. Außerdem besitzen von den synthetischen anionischen Tensiden die Sulfate und Sulfonate praktische Bedeutung. Zu diesen Produkten gehören z. B. die Alkylarylsulfonate und aliphatischen Sulfonate wie z. B. Alkansulfonate, Alkensulfonate, Hydroxyalkansulfonate und Hydroxyalkensulfonate, außerdem Fettalkoholsulfate und Sulfatierungsprodukte von oxalkylierten Alkylphenolen, Fettsäureamiden oder Fettsäurealkylolamiden mit einem Gehalt von etwa 1 bis 20 Ethoxy- und/oder Propoxyresten im Molekül sowie sulfatierte Monoglyceride. Die für die Verwendung in Waschmitteln geeigneten anionischen waschaktiven Substanzen sind ausführlich z. B. in « Surface Active Agents and Detergents » von Schwartz, Perry and Berch, Vol. II (1958), S. 25 bis 102, beschrieben.

Als nichtionische waschaktive Verbindungen werden solche Produkte, die ihre Wasserlöslichkeit des hydrophoben Molekülanteils durch die Anwesenheit von Polyetherketten, Aminoxid-, Sulfoxid- oder Phosphinoxidgruppen, Alkylolamidgruppierungen oder eine Häufung von Hydroxylgruppen verdanken, verwendet.

Von besonderem praktischen Interesse sind die durch Anlagerung von Ethylenoxid und/oder Glycid an Fettalkohole, Alkylphenole, Fettsäuren, Fettamine, Fettsäure- oder Sulfonsäureamide erhältlichen Produkte, welche normalerweise 6-40 und vorzugsweise 8-20 Etherreste, vor allem Ethylenglykoletherreste, pro Molekül enthalten können. Weitere Einzelheiten über die nichtionischen Tenside werden z. B. in « Suface Active Agents and Detergents » Vol II (1958), S. 120-143, beschrieben.

Zu den zwitterionischen Tensiden gehören solche Verbindungen, die neben einem hydrophoben Alkylrest mit vorzugsweise 10-20 C-Atomen sowohl saure als auch basische hydrophile Gruppen enthalten. Zu den sauren Gruppen gehören Carboxyl-, Sulfonsäure-, Schwefelsäurehalbester-, Phosphonsäure- und Phosphorsäureteilestergruppen. Als basische Gruppen kommen primäre, sekundäre, tertiäre und quaternäre Ammoniumgruppen in Frage. Solche zwitterionischen Verbindungen mit quaternären Ammoniumgruppen gehören zum Typ der Betaine. Wegen ihrer guten Verträglichkeit mit anderen Tensiden haben Carboxy-Sulfat- und Sulfonatbetaine besonders praktisches Interesse.

Beispiele solcher zwitterionischen Verbindungen sind N-Alkyl-beta-aminopropionsäure, N-Alkyl-beta-iminodipropionsäure, N-Alkyl-N,N-dimethylglycin bzw. deren Salze insbesonders Natriumsalze und 1-Alkyl-5-hydroxyethyl-5-carboxymethylimidazolin. Die Alkylreste leiten sich z. B. von Stearyl-, Lauryl-, Kokosfett-, Myristyl- oder Cetylalkohol bzw. von deren technischen Gemischen ab. Weitere Beispiele solcher zwitterionischer Tenside sind Sulfobetaine, welche durch Umsetzen von tertiären Aminen mit Sultonen erhalten werden und Carboxybetaine, welche durch Umsetzen von tertiären Aminen mit Chloressigsäure oder deren Salze erhalten werden, wobei die tertiären Amine wenigstens einen hydrophoben Alkylrest enthalten müssen.

Zu den kationischen Tensiden gehören solche Verbindungen, die ihre Wasserlöslichkeit des hydrophoben Molekülanteils durch die Anwesenheit von kationischen wasserlöslichmachenden Gruppen verdanken. Solche kationisch, löslichmachende Gruppen sind Amine und quaternäre Gruppen.

Von besonderem praktischen Interesse sind dabei N-Alkylethylendiamin, wobei der Alkylrest 12 bis 22 C-Atome aufweisen kann, wie z. B. N-2-Amino-ethyl-stearylamin oder N-Myristyl-ethylendiamin, sowie 2-Aminoethyl-carbonsäureamide, wobei die Carbonsäureamide von aliphatischen $C_{10}$ bis $C_{20}$ Carbonsäuren abgeleitet sind, wie z. B. N-2-Aminoethylstearylamid oder N-2-Aminoethyl-myristylamid. Typische quaternäre Ammoniumverbindungen sind z. B. Ethyldimethyl-stearylammoniumchlorid, Trimethyl-cetylammoniumbromid, Benzyl-dimethyl-stearylammoniumchlorid, Benzyl-diethyl-stearylammoniumchlorid, Trimethyl-stearyl-ammoniumchlorid, Dimethyl-ethyl-laurylammoniumchlorid, Dimethyl-propyl-myristylammoniumchlorid sowie die entsprechenden Acetate und Methosulfate.

Das Schaumvermögen der Tenside läßt sich durch Kombination geeigneter Tensidtypen steigern oder verringern, ebenso wie es durch Zusätze nicht tensidartiger organischer Substanzen verändert werden kann.

Das Schaumvermögen der synthetischen anionischen oder nichtionischen Tenside läßt sich durch Zusatz von Seifen, insbesondere mit solchen Seifen, die einen hohen Anteil an $C_{20}$-$C_{24}$-Fettsäureanteilen enthalten, verringern. Durch Kombinationen aus gewissen synthetischen anionischen Tensiden, nichtionischen Tensiden und Seifen kann das Schaumvermögen reguliert werden. Ferner zeichnen sich die

Anlagerungsverbindungen von Propylenoxid an oberflächenaktive Polyethylenglykolether durch ein geringes Schaumvermögen aus.

Eine weitere wesentliche Gruppe der Waschmittel stellen die Gerüstsubstanzen oder sogenannten Builder dar. Dazu eignen sich schwach sauer, neutral oder alkalisch reagierende anorganische oder organische Salze, insbesondere anorganische oder organische Komplexbildner. Von diesen soll wenigstens ein Teil eine alkalische Reaktion aufweisen.

Brauchbare Gerüstsubstanzen sind z. B. die Hydrogencarbonate, Carbonate, Silikate oder Citrate der Alkalimetalle, weiterhin Mono-, Di- oder Trialkaliorthophosphate, Di- oder Tetraalkalipyrophosphate sowie die als Komplexbildner bekannten Metaphosphate. Als Builder sind ferner die wasserlöslichen Salze höhermolekularer Polycarbonsäuren brauchbar. Als solche kommen vor allem Polymerisate der Maleinsäure, Fumarsäure, Itaconsäure, Mesaconsäure, Aconitsäure und Methylenmalonsäure in Betracht. Auch Mischpolymerisate dieser Säuren untereinander oder mit anderen polymerisierbaren Stoffen wie Ethylen, Propylen, Acrylsäure, Methacrylsäure, Crotonsäure, Vinylacetat, Acrylamid und Styrol sind geeignet.

Als komplexbildende Gerüstsubstanzen eignen sich vor allem die schwach sauer reagierenden Metaphosphate sowie die alkalisch reagierenden Polyphosphate, insbesondere das Tripolyphosphat. Zu den organischen Komplexbildern gehören beispielsweise Nitrilotriessigsäure, Ethylendiaminotetraessigsäure, N-Hydroxyethyl-ethylendiamintriessigsäure und ähnliche Verbindungen. Weitere geeignete anorganische und organische Gerüstsubstanzen werden beschrieben in « Surface Active Agents and Detergents » Vol. II (1958) Seiten 289 bis 317.

Den Mischungen aus den erfindungsgemäßen Aktivatoren und den Perverbindungen können gegebenenfalls Produkte zugesetzt werden, die stabilisierend auf die Perverbindungen wirken. Bei diesen Verbindungen, die unter dem Begriff Stabilisatoren für Perverbindungen bekannt sind, kann es sich um wasserlösliche oder wasserunlösliche Produkte handeln, welche in Mengen bis zu 10 Gew.-%, bezogen auf Perverbindungen, zugesetzt werden können.

Als wasserunlösliche Perstabilisatoren eignen sich vor allem Erdalkalisilikate, insbesondere Magnesiumsilikate, welche meist durch Fällung aus wäßrigen Alkalisilikatlösungen mit Erdalkalisalzen erhalten werden. Weitere wasserunlösliche Perstabilisatoren stellen z. B. wasserhaltige Zinnoxide dar.

Als wasserlösliche Perstabilisatoren, die ganz oder teilweise anstelle der wasserunlöslichen Perstabilisatoren verwendet werden können, kommen vor allem organische Komplexbildner in Betracht.

Den Mischungen aus den erfindungsgemäßen Aktivatoren und den Perverbindungen können neben den Tensiden und den Gerüststoffen noch weitere Hilfs- und Zusatzstoffe zugesetzt werden.

Von diesen Hilfs- oder Zusatzstoffen können weiterhin Schmutzträger enthalten sein, die den von der Faser abgelösten Schmutz in der Waschflotte suspendiert halten. Dazu sind wasserlösliche Kolloide meist organischer Natur geeignet, wie z. B. die Salze polymerer Carbonsäuren, Leim, Gelatine, Salze von Ethercarbonsäure, Carboxymethylcellulose, Stärke oder Polyvinylpyrrolidon.

Von den weiterhin zu verwendenden optischen Aufheller sind besonders die Derivate der Diaminostilbensulfonsäure, der Diarylpyrazoline und der Aminocumarine zu erwähnen.

Beispiele für Aufheller aus der Klasse der Diaminostilbensulfonsäurederivate sind Verbindungen gemäß Formel III

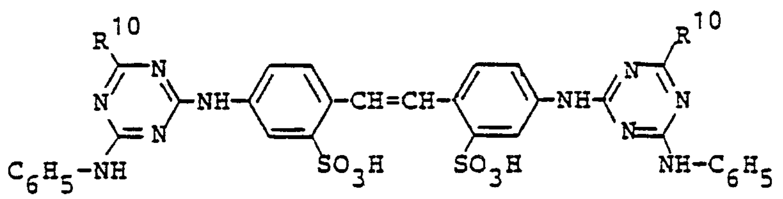

(III)

worin $R^{10}$ folgende Reste bedeuten kann :

$-NH_2$, $-NH-CH_3$, $-NH-CH_2-CH_2-OH$, $-NH-CH_2-CH_2-OCH_3$, $-NH-CH_2-CH_2-CH_2-OCH_3$, $CH_3-N-CH_2-CH_2-OH$, $-N=(CH_2-CH_2-OH)_2$

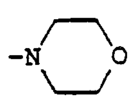, $-NH-C_6H_5$, $-NH-C_6H_4SO_3H$, $-OCH_3$.

Insbesondere bedeutet der Rest $R^{10}$ $-NH-CH_3$ bzw.

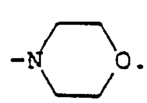

Eine weitere Verbindung von Diaminostilbensulfonsäuretyp ist die folgende Bis-triazolylstilbendisulfonsäure :

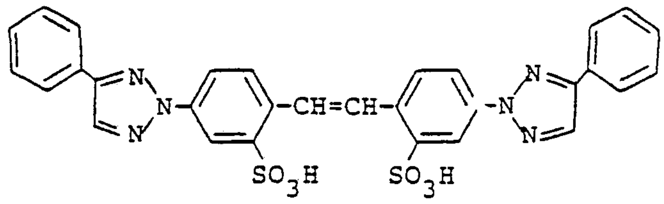

Eine weitere Gruppe von Aufhellern sind die Distyrylbisphenyl-disulfonsäuren der allgemeinen Formel IV

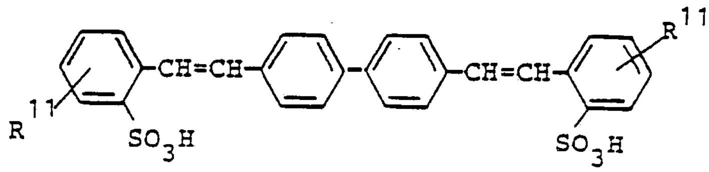

(IV)

worin $R^{11}$ bevorzugt Wasserstoff und/oder Chlor bedeutet.

Eine weitere Gruppe der optischen Aufheller sind die Diarylpyrazoline, von denen vorzugsweise solche der allgemeinen Formel V

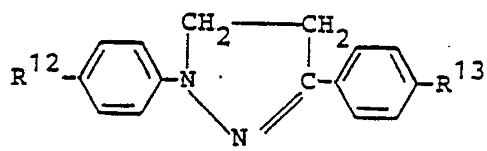

(V)

wobei bei den im Handel befindlichen Aufhellern $R^{13}$ insbesondere Cl und $R^{12}$ insbesondere Cl, $-SO_2-NH_2$, $-SO_2-CH=CH_2$ und $-COO-CH_2-OCH_3$ darstellen kann, verwendet werden.

Zu den Aufhellern gehören weiterhin aliphatische oder aromatische substituierte Aminocumarine, z. B. das 4-Methyl-7-dimethylaminocumarin oder das 4-Methyl-7-diethylaminocumarin.

Als Hilfs- oder Zusatzstoffe zu den erfindungsgemäßen Bleich- oder bleichenden Waschmitteln können außerdem Enzyme, meist Gemische verschiedener enzymatischer Wirkstoffe, z. B. Proteasen, Lipasen, Ureasen, Amylasen, verwendet werden.

Die erfindungsgemäßen Bleich- oder bleichenden Waschmittel können ferner Farbstoffe, Duftstoffe, Hydrotrope Substanzen sowie Wasser, insbesondere als Kristallwasser, enthalten.

Die Herstellung sowie die Verwendung der erfindungsgemäßen Aktivatoren der allgemeinen Formel I wird durch die nachfolgenden Beispiele erläutert. In diesen Beispielen bedeuten die Prozentangaben, falls nicht ausdrücklich etwas anderes angegeben ist, Gewichtsprozente.

## Beispiele

### Herstellen der acylierten Triazolidin-3,5-dione :

1) 1,2-Bisacetyl-triazolidin-3,5-dion

Eine Mischung aus 101 g Triazolidin-3,5-dion und 225 g Acetanhydrid wird auf 130 °C erhitzt und nach dem Abklingen der leicht exothermen Reaktion 30 Minuten bei 130 °C gerührt. Nach dem Abkühlen wird abgesaugt, der Rückstand wird mit Essigsäure und Ethanol gewaschen und bei 30 mbar und 60 °C getrocknet. Es werden 150 g (= 81 % der Theorie) 1,2-Bisacetyltriazolidin-3,5-dion vom Fp. = 203-205 °C erhalten.

$C_6H_7N_3O_4$ berechnet : C = 38,93 % H = 3,81 % N = 22,69 %
(185,1) gefunden : C = 38,9 % H = 3,7 % N = 22,8 %

2) 1,2,4-Trisacetyl-triazolidin-3,5-dion

Eine Mischung aus 185 g 1,2-Bisacetyl-triazolidin-3,5-dion und 1 kg Acetanhydrid wird 8 Stunden bei leichtem Rückfluß gerührt. Ein Teil des Lösungsmittels wird abdestilliert. Nach dem Abkühlen wird abgesaugt, mit Essigsäure und dann mit Essigsäureethylester gewaschen und bei 60 °C und 30 mbar getrocknet. Es werden 152 g (= 67 % der Theorie) 1,2,4-Trisacetyltriazolidin-3,5-dion vom Fp. = 137-138 °C erhalten.

$C_8H_9N_3O_5$ berechnet : C = 42,29 % H = 4,00 % N = 18,50 %
(227,2) gefunden : C = 42,2 % H = 3,9 % N = 18,6 %

3) 1,2-Ethan-diyl-4,4'-bis-[1,2-bisacetyl-triazolidin-3,5-dion]

Eine Mischung aus 456 g 1,2-Ethan-diyl-4,4'-bis-triazolidin-3,5-dion, 50 g Pyridin und 100 g Acetanhydrid wird auf 130 °C erwärmt und 1 Stunde bei 130 °C gerührt. Nach dem Abkühlen wird der entstandene kirstalline Niederschlag abgesaugt, mit Essigsäure und dann mit Essigsäureethylester gewaschen und bei 60 °C und 30 mbar getrocknet. Es werden 75 g (= 95 % der Theorie) 1,2-Ethan-diyl-4,4'-bis-[1,2-bisacetyl-triazolidin-3,5-dion] vom Fp. = 226-227 °C erhalten, dessen Struktur durch IR- und NMR-Spektren sowie der Elementaranalyse bestätigt wird.

$C_{14}H_{16}N_6O_8$ berechnet : C = 42,43 % H = 4,07 % N = 21,21 %
(396,3) gefunden : C = 42,5 % H = 4,1 % N = 21,1 %

4) 1,2-Bispropionyl-1,2,4-triazolidin-3,5-dion

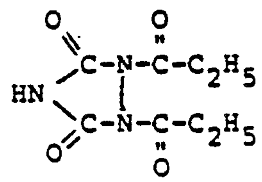

Zu 101 g 1,2,4-Triazolidin-3,5-dion in 100 g Pyridin werden bei 100 °C innerhalb von 2 Stunden 270 g Propionsäureanhydrid getropft. Zur Vervollständigung der Reaktion wird noch 1 Stunde bei 100 °C gerührt. Nach dem Abkühlen auf Raumtemperatur wird der Niederschlag abgesaugt, zuerst mit Propionsäure und dann mit Ethanol gewaschen und bei 70 °C im Vakuum getrocknet. Es werden 187 g (= 88 % der Theorie) 1,2-Bispropionyl-1,2,4-triazolidin-3,5-dion vom Fp = 208 °C erhalten.

$C_8H_{11}N_3O_4$ berechnet : C = 45,07 % H = 5,20 % N = 19,71 %
(213,2) gefunden : C = 44,9 % H = 5,1 % N = 20,0 %

5) 1,2-Bisacetyl-4-phenyl-triazolidin-3,5-dion

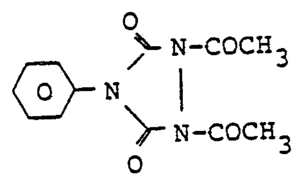

Zu 177 g 4-Phenyl-triazolidin-3,5-dion in 100 g Pyridin werden bei 100 °C innerhalb von 30 Minuten 240 g Acetanhydrid getropft. Zur Vervollständigung der Reaktion wird 1 Stunde bei 100 °C nachgerührt. Die heiße Lösung wird mit 500 ml Toluol versetzt, abgekühlt und der Niederschlag abgesaugt. Es werden 192 g 1,2-Bisacetyl-4-phenyl-triazolidin-3,5-dion vom Fp = 165-166 °C (aus Ethanol) erhalten. Aus der Mutterlauge kirstallisieren nach Zugabe von 150 ml Petroläther noch 37 g der gewünschten Verbindung aus. Durch IR- und NMR-Spektren sowie der Elementaranalyse wird die angenommene Struktur bestätigt.

$C_{12}H_{11}N_3O_4$ berechnet : C = 55,17 % H = 4,25 % N = 16,09 %
(261,2) gefunden : C = 55,1 % H = 4,1 % N = 16,2 %

6) 1,2-Bis-(2-methyl-ethylcarbonyl)-4-phenyl-triazolidin-3,5-dion

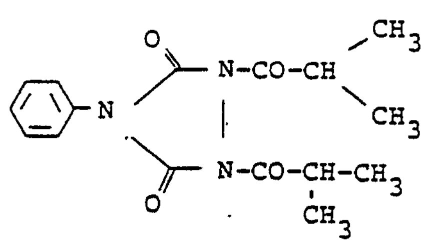

Zu 17,7 g 4-Phenyl-triazolidin-3,5-dion und 20 g Pyridin werden bei 100 °C 33 g Isobuttersäureanhydrid getropft. Die klare Lösung wird 1 Stunde bei 100 °C gerührt. Das Lösungsmittel wird sodann im Wasserstrahlvakuum abdestilliert und der Rückstand mit 40 ml Toluol versetzt. Der Niederschlag wird abgesaugt, mit Toluol gewaschen und getrocknet. Es werden 21 g 1,2-Bis-(2-methyl-ethylcarbonyl)-4-phenyl-triazolidin-3,5-dion vom Fp = 104-105 °C erhalten, dessen Struktur mittels IR- und NMR-Spektren sowie der Elementaranalyse bestätigt wird.

$C_{16}H_{19}N_4O_4$ berechnet : C = 60,56 % H = 6,04 % N = 13,24 %
(317,3) gefunden : C = 60,7 % H = 6,1 % N = 13,4 %

7) 1,2-Bisacetyl-4-n-stearyl-triazolidin-3,5-dion :

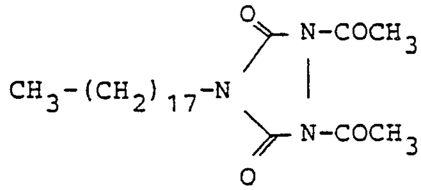

Zu 353 g 4-n-Stearyl-triazolidin-3,5-dion und 1 kg Essigsäure werden bei 120 °C innerhalb 2 Stunden 255 g Acetanhydrid getropft. Zur Vervollständigung der Reaktion wird 3 Stunden bei Rückfluß gerührt. Nach dem Abkühlen wird der kristalline Niederschlag abgesaugt, mit Essigsäure und Essigsäureethylester gewaschen und getrocknet. Es werden 290 g 1,2-Bisacetyl-4-n-stearyl-triazolidin-3,5-dion vom Fp = 100-101 °C (aus Essigsäureethylester) erhalten, dessen Struktur mittels IR- und NMR-Spektren sowie der Elementaranalyse bestätigt wird.

$C_{24}H_{43}N_3O_4$ berechnet : C = 65,87 % H = 9,90 % N = 9,60 %
(437,6) gefunden : C = 66,1 % H = 10,1 % N = 9,8 %

8) 4,4′-Bis-[(1,2-bisacetyl)-triazolidin-3,5-dion-4-yl]-dicyclohexylmethan

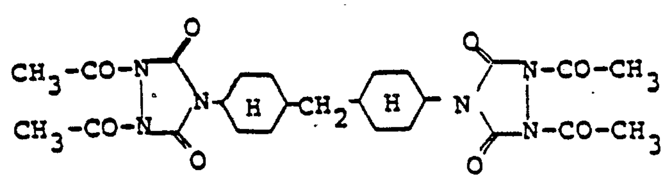

Zu 37,8 g 4,4′-Bis-(triazolidin-3,5-dion-4-yl)-dicyclohexylmethan und 50 g Essigsäure werden bei Rückfluß innerhalb 30 Minuten 50 g Acetanhydrid getropft. Zur Vervollständigung der Reaktion wird 2 Stunden bei Rückfluß gerührt. Aus der klaren Lösung wird im Vakuum ein Teil des Lösungsmittels abdestilliert und der Rückstand mit 100 ml Dioxan verrührt. Nach dem Abkühlen wird der Niederschlag abgesaugt, mit Dioxan gewaschen und getrocknet. Es werden 29,5 g 4,4′-Bis-(1,2-bisacetyl)-triazolidin-3,5-dion vom Fp = 252-253 °C erhalten, dessen Struktur mittels IR- und NMR-Spektren sowie der Elementaranalyse bestätigt wird.

$C_{25}H_{34}N_6O_8$ berechnet : C = 54,93 % H = 6,27 % N = 15,38 %
(546,6) gefunden : C = 54,7 % H = 6,1 % N = 15,5 %

9) 1,2 Bisacetyl-4-methyl-triazolidin-3,5-dion

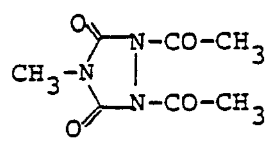

Zu 115 g 4-Methyl-triazolidin-3,5-dion und 150 g Essigsäure werden bei Rückfluß innerhalb einer Stunde 250 g Essigsäureanhydrid getropft. Zur Vervollständigung der Reaktion wird die entstandene klare Lösung 3 Stunden bei Rückfluß gerührt. Das Lösungsmittel wird im Vakuum größtenteils abdestilliert und der Rückstand aus 300 ml Dioxan umkristallisiert. Der Niederschlag wird abgesaugt, mit Dioxan gewaschen und getrocknet. Es werden 108 g 1,2-Bisacetyl-4-methyl-triazolidin-3,5-dion vom Fp = 138 °C erhalten, dessen Struktur mittels IR- und NMR-Spektren sowie der Elementaranalyse bestätigt wird.

$C_7H_9N_3O_4$ berechnet : C = 42,20 % H = 4,55 % N = 21,10 %
(199,2) gefunden : C = 42,0 % H = 4,4 % N = 21,2 %

10) 1,2-Bisacetyl-4-cyclohexyl-triazolidin-3,5-dion :

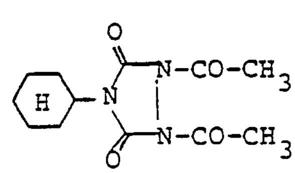

Zu 91,5 g 4-Cyclohexyl-triazolidin-3,5-dion und 100 g Essigsäure werden bei 110 °C innerhalb einer Stunde 125 g Acetanhydrid getropft. Die klare Lösung wird 3 Stunden bei Rückfluß gerührt. Ein Teil des Lösungsmittels wird im Vakuum abdestilliert und der Rückstand mit Cyclohexan verrührt. Nach dem Abkühlen wird abgesaugt, mit Cyclohexan gewaschen und getrocknet. Es werden 78,5 g 1,2-Bisacetyl-4-cyclohexyl-triazolidin-3,5-dion vom Fp = 126-127 °C erhalten, dessen Struktur mittels IR- und NMR-Spektren sowie der Elementaranalyse bestätigt wird.

$C_{12}H_{17}N_3O_4$ berechnet : C = 53,92 % H = 6,41 % N = 15,72 %
(267,3) gefunden : C = 54,1 % H = 6,5 % N = 15,6 %

## Beispiele 11 bis 17

a) Durch Sprühtrocknen wurde ein schaumarmes Waschpulver der folgenden Zusammensetzung hergestellt :

12,0 % anionisches Tensid (= Alkylbenzolsulfonat-Na-Salz)
4,7 % nichtionisches Tensid (Fettalkoholpolyglykolether mit 11 Mol Ethylenoxid)
4,0 % Seife als Schraumregulator
6,7 % Natriumsilikat ($Na_2SiO_3$)
2,0 % Carboxymethylcellulose
19,0 % Natriumsulfat
40,0 % Natriumtripolyphosphat
0,3 % Weißtöner
ca. 11,3 % Restfeuchte

b) Durch anschließendes Zumischen der übrigen Bestandteile wurden Waschmittel hergestellt, deren Zusammensetzungen aus der folgenden Tabelle 1 ersichtlich sind :

Tabelle 1

| Beispiel | 11 % | 12 % | 13 % | 14 % | 15 % | 16 % | 17 % |
|---|---|---|---|---|---|---|---|
| Waschpulver schaumarm gesteuert | 74,7 | 74,7 | 74,7 | 77,0 | 74,7 | 75,9 | 74,7 |
| Natriumperborattetrahydrat | 22,0 | 15,0 | 15,0 | 15,0 | 15,0 | 15,0 | 15,0 |
| Magnesiumsilikat | 3,0 | 3,0 | 3,0 | 3,0 | 3,0 | 3,0 | 3,0 |
| Ethylendiamintetraessig-säure-Na-Salz | 0,3 | 0,3 | 0,3 | 0,3 | 0,3 | 0,3 | 0,3 |
| Tetraacetylethylendiamin | - | 7,0 | - | - | - | - | - |
| Triazolidin nach Beispiel 2 | - | - | 7,0 | 4,7 | - | - | - |
| Triazolidin nach Beispiel 1 | - | - | - | - | 7,0 | 5,8 | - |
| Triazolidin nach Beispiel 3 | - | - | - | - | - | - | 7,0 |

c) Bleichtestgewebe Immedialgrün WFK 10 E wurden mit diesen Waschmitteln aus Tabelle 1 bei einer Waschmittelkonzentration von 5 g/Liter und einem Flottenverhältnis von 1 : 20 in Wasser von 18° dH 30 Minuten bei 40 °C bzw. 30 Minuten bei 60 °C gewaschen. Der Waschprozeß wurde in einem Launder-o-meter durchgeführt.

Die bei diesen Waschversuchen erzielten Aufhellungen wurden durch Remissionsmessungen mit UV-Sperrfilter bei 460 nm am RFC 3 der Firma Zeiss nach DIN 44 983 Blatt 1, Seite 3, wie von der Bundesanstalt für Materialprüfung/Berlin beschrieben, durchgeführt. Die Ergebnisse dieser Untersuchungen sind in der Tabelle 2 zusammengefaßt.

(Siehe Tabelle 2 Seite 13 f.)

Tabelle 2

| Waschmittel gemäß Beispiel | R-Werte bei $40^{\circ}C$, 4 x gewaschen | bei $60^{\circ}C$, 4 x gewaschen |
|---|---|---|
| 11 | 33,2 | 36,7 |
| 12 | 37,8 | 42,3 |
| 13 | 38,7 | 46,8 |
| 14 | 37,4 | 42,7 |
| 15 | 38,0 | 45,2 |
| 16 | 36,6 | 44,0 |
| 17 | 37,6 | 43,5 |

Die obigen Ergebnisse zeigen, daß die Waschmitelformulierungen mit den erfindungsgemäßen Aktivatoren gegenüber der Waschmittelformulierung ohne Aktivator eine erheblich bessere Aufhellung erreichen und daß die Waschmittelformulierungen mit den erfindungsgemäßen Aktivatoren gegenüber der Waschmittelformulierung mit dem bekannten und allgemein verwendeten Aktivator Tetraacetylendiamin bei gleicher Konzentration eine bessere Aufhellung erzielen.

**Patentansprüche**

1. Verwendung von Verbindungen der Formel

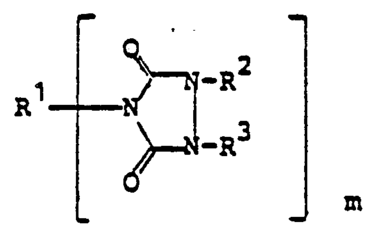

(I)

in der

$R^1$ Wasserstoff oder einen organischen Rest bedeutet, wobei die Bindung $R^1$-N eine C-N-Bindung ist;

$R^2$, $R^3$ für Wasserstoff oder einen organischen Rest stehen, wobei die Bindungen $R^2$-N und $R^3$-N C-N-Bindungen sind, und

m 1, 2, 3, 4 oder 5 bedeutet,

mit der Maßgabe, daß mindestens einer der Reste $R^1$, $R^2$ oder $R^3$ für eine Acylgruppe steht, als Aktivatoren für Perverbindungen.

2. Verwendung von Verbindungen der Formel I, in der $R_2$, $R_3$ und m die in Anspruch 1 angegebenen Bedeutungen haben, und in der sich der Rest $R^1$ von einem $C_1$-$C_{20}$-Alkan, einem $C_3$-$C_7$-Cycloalkan, Benzol, Naphthalin, einem Phenyl-$C_1$-$C_6$-alkan, einem Kohlenwasserstoff des Typs

$$X_1—(C_nH_{2n}—X_2)_n—X_3$$

wobei $X_1$, $X_2$, $X_3$ Cyclopentyl, Cyclohexyl oder Phenyl bezeichnen, wobei $X_1$, $X_2$ und $X_3$ vorzugsweise die gleiche Bedeutung haben und n 1, 2, 3 oder 4 bezeichnet, einem aliphatischen Ether mit 2-20 C-Atomen, einem aromatischen Ether mit 12-24 C-Atomen, einem gemischt aliphatisch-aromatischen Ether mit 7-24 C-Atomen, einem aliphatischen tertiären Amin mit 3-20 C-Atomen oder einem aromatischen tertiären Amin mit 8-24 C-Atomen ableitet, wobei weiterhin die für $R^1$ genannten Kohlenwasserstoffreste z. B. durch ($C_1$-$C_4$-Alkoxy)-carbonyl, CN, Halogen und im Falle aromatischer Reste auch durch Niedrigalkyl substituiert sein können, als Aktivatoren für Perverbindungen.

3. Verwendung von Verbindungen der Formel I, in der $R_2$, $R_3$ und m die in Anspruch 1 angegebenen Bedeutungen haben, und bei der $R^1$ für einen Rest $C_nH_{2n+1}$ mit n = 1-12, $—(CH_2)_m—$ mit m = 2-12,

$-[CH_2-\underset{R^8}{CH}-O]_p-CH_2-CH-$ mit $R^8$ = H, $CH_3$ und p = 1 bis 9,

$-[CH_2-(CH_2)_q-\underset{R^9}{N}-]_r(CH_2)_q-CH_2-$ mit $R^9$ = $C_1$-$C_4$-Alkyl,

q = 1, 2 und r = 1-4,

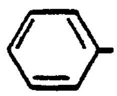 , 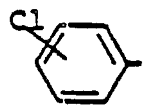 , 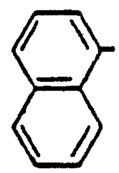 , 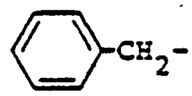 , 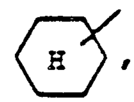 ,

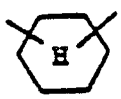 , 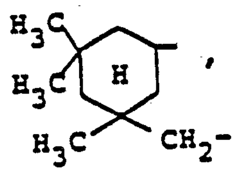 , 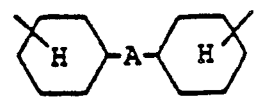

mit A = Alkylen mit 1-4 C-Atomen,

$-O-$ oder $-\underset{CH_3}{N}-$

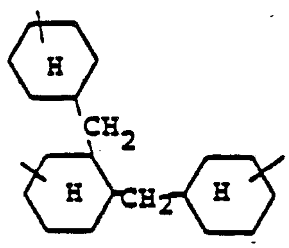 , 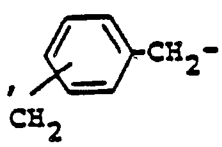 , 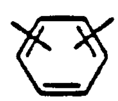 , 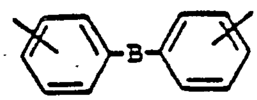

mit B = Alkylen mit 1-4 C-Atomen,

$-O-$ oder $-\underset{CH_3}{N}-$ ,

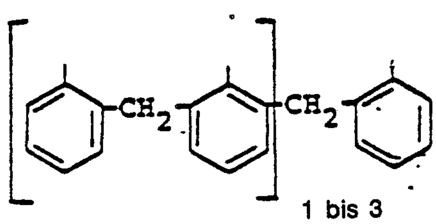

1 bis 3

$C_4H_9-O-CH_2CH_2CH_2-$,

$-(CH_2)_3-O-(CH_2)_{2-4}-O-(CH_2)_3-$,

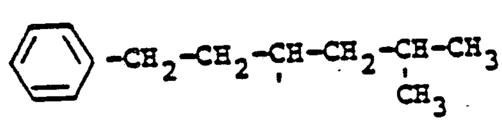

Wasserstoff,

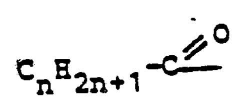

mit n = 1-3 oder

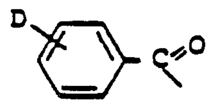

mit D = H, Cl, $CH_3$, $NO_2$, steht, als Aktivatoren für Perverbindungen.

4. Verwendung von Verbindungen der Formel I, in der R, und m die in Anspruch 1 angegebenen Bedeutungen haben, und in der $R^2$, $R^3$ für ($C_1$-$C_8$-Alkyl)-carbonyl, Benzoyl oder Phenyl-($C_1$-$C_4$-alkyl)-carbonyl stehen, wobei die Acylreste weitere Substituenten wie beispielsweise $C_1$-$C_4$-Alkoxy, Halogen, Nitro und Cyan tragen können, als Aktivatoren für Perverbindungen.

5. Verwendung von Verbindungen der Formel I, bei denen R, die in Anspruch 1 angegebene Bedeutung hat, m = 1 oder 2 und $R^2 = R^3$ = Acetyl, Propionyl oder Benzoyl oder m = 1 und $R^1 = R^2 = R^3$ = Acetyl, Propionyl oder Benzoyl sind, als Aktivatoren für Perverbindungen.

6. Bleich- und/oder Waschmittel enthaltend Verbindungen gemäß den Ansprüchen 1-5 und eine Perverbindung.

7. Bleich- und/oder Waschmittel gemäß Anspruch 6, dadurch gekennzeichnet, daß sie die Verbindungen gemäß den Ansprüchen 1-5 in einer solchen Menge enthalten, daß 0,2 bis 6 abspaltbare Acylreste pro Aktivsauerstoffatom der Perverbindung vorliegen.

8. Bleich- und/oder Waschmittel, dadurch gekennzeichnet, daß diese 5-50 Gew.-% Perverbindung und Aktivator gemäß den Ansprüchen 1-5, 5-40 Gew.-% Tenside, 10-80 Gew.-% Gerüststoffe und 0-15 Gew.-% sonstige Hilfs- und Zusatzstoffe enthalten.

**Claims**

1. Use of compounds of the formula

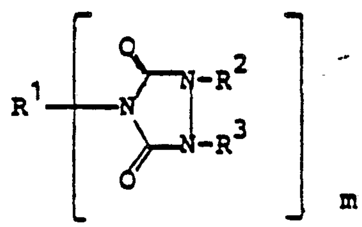

(I)

in which

$R^1$ denotes hydrogen or an organic radical, the bond $R^1$-N being a C-N bond ;

$R^2$ and $R^3$ represent hydrogen or an organic radical, the bonds $R^2$-N and $R^3$-N being C-N bonds, and

m denotes 1, 2, 3, 4 or 5,

with the proviso that at least one of the radicals $R^1$, $R^2$ or $R^3$ represents an acyl group, as activators for per compounds.

2. Use of compounds of the formula I in which $R_2$, $R_3$ and m have the meanings given in Claim 1, and in which the radical $R^1$ is derived from a $C_1$-$C_{20}$-alkane, a $C_3$-$C_7$-cycloalkane, benzene, naphthalene, a phenyl-$C_1$-$C_6$-alkane, a hydrocarbon of the type

$$X_1—(C_nH_{2n}—X_2)_n—X_3$$

wherein $X_1$, $X_2$ and $X_3$ designate cyclopentyl, cyclohexyl or phenyl, $X_1$, $X_2$ and $X_3$ preferably having the same meaning, and n designates 1, 2, 3 or 4, an aliphatic ether with 2-20 C atoms, an aromatic ether with 12-24 C atoms, a mixed aliphatic-aromatic ether with 7-24 C atoms, an aliphatic tertiary amine with 3-20 C atoms or an aromatic tertiary amine with 8-24 C atoms, it furthermore being possible for the hydrocarbon radicals mentioned for $R^1$ to be substituted, for example by ($C_1$-$C_4$-alkoxy)-carbonyl, CN, halogen and, in the case of aromatic radicals, also by lower alkyl, as activators for per compounds.

3. Use of compounds of the formula I, in which $R_2$, $R_3$ and m have the meanings given in Claim 1 and in which $R^1$ represents a radical $C_nH_{2n+1}$, in which n = 1-12, $—(CH_2)_m—$, in which m = 2-12,

$-[CH_2-\underset{R^8}{CH}-O]_p-CH_2-CH-$ in which $R^8$ = H or $CH_3$ and p = 1 to 9,

$-[CH_2-(CH_2)_q-\underset{R^9}{N}-]_r(CH_2)_q-CH_2-$ in which $R^9$ = $C_1$-$C_4$-Alkyl,

q = 1, 2 and r = 1-4,

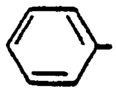 , 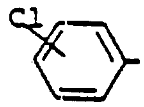 , 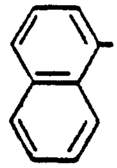 , 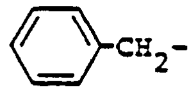 , 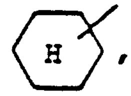 ,

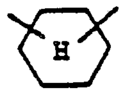 , 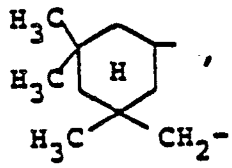 , 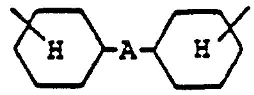

in which A = alkylene with 1-4 C atoms,

$-O-$ or 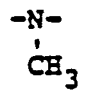 ,

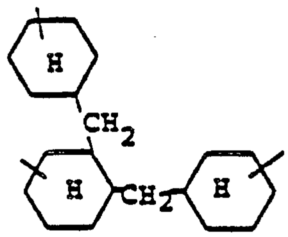 , 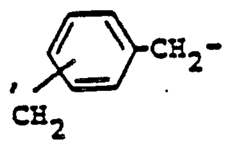 , 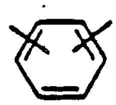 , 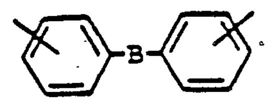

in which B = alkylene with 1-4 C atoms,

$-O-$ or 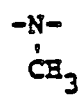 ,

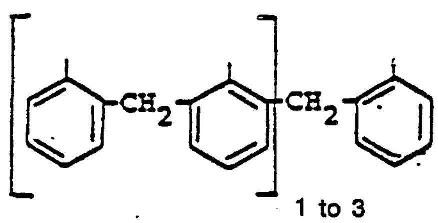
1 to 3

$C_4H_9-O-CH_2CH_2CH_2-$,

$-(CH_2)_3-O-(CH_2)_{2\text{-}4}-O-(CH_2)_3-$,

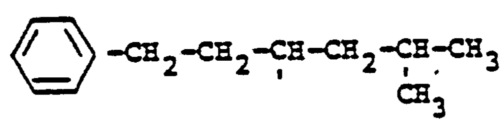

hydrogen,

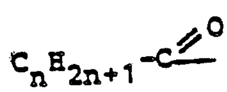

in which n = 1-3 or

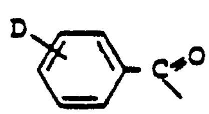

in which D = H, Cl, $CH_3$ or $NO_2$, as activators for per compounds.

4. Use of compounds of the formula I in which $R_1$ and m have the meanings given in Claim 1 and in

which $R^2$ and $R^3$ represent ($C_1$-$C_8$-alkyl)-carbonyl, benzoyl or phenyl-($C_1$-$C_4$-alkyl)-carbonyl, it being possible for the acyl radicals to carry further substituents such as for example $C_1$-$C_4$-alkoxy, halogen, nitro and cyano, as activators for per compounds.

5. Use of compounds of the formula I, in which $R_1$ has the meaning given in Claim 1, m = 1 or 2 and $R^2 = R^3$ = acetyl, propionyl or benzoyl or m = 1 and $R^1 = R^2 = R^3$ = acetyl, propionyl or benzoyl, as activators for per compunds.

6. Bleaching agents and/or detergents containing compounds according to Claims 1-5 and a per compound.

7. Bleaching agents and/or detergents according to Claim 6, characterised in that they contain the compounds according to Claims 1-5 in such a quantity that 0.2 to 6 cleavable acyl radicals are present per active oxygen atom of the per compound.

8. Bleaching agents and/or detergents, characterised in that they contain 5-50 % by weight of the per compound and activator according to Claims 1-5, 5-40 % by weight of surfactants, 10-80 % by weight of builders and 0-15 % by weight of other auxiliaries and additives.

**Revendications**

1. Utilisation de composés de formule

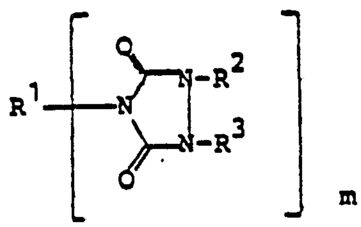

(I)

dans laquelle

$R^1$ désigne l'hydrogène ou un reste organique, la liaison $R^1$-N étant une liaison carbone-à-azote :

$R^2$, $R^3$ représentent l'hydrogène ou un reste organique, les liaisons $R^2$-N et $R^3$-N étant des liaisons carbone-à-azote et

m a la valeur 1, 2, 3, 4 ou 5,

sous réserve qu'au moins l'un des restes $R^1$, $R^2$ ou $R^3$ soit un groupe acyle, comme activateurs pour percomposés.

2. Utilisation de composés de formule I dans laquelle $R_2$, $R_3$ et m ont les définitions indiquées dans la revendication 1 et dans laquelle le reste $R^1$ dérive d'un alcane en $C_1$ à $C_{20}$, d'un cycloalcane en $C_3$ à $C_7$, du benzène, du naphtalène, d'un phénylalcane en $C_1$ à $C_6$, d'un hydrocarbure du type

$$X_1-(C_nH_{2n}-X_2)_n-X_3$$

où $X_1$, $X_2$, $X_3$ représentent un groupe cyclopentyle, cyclohexyle ou phényle, $X_1$, $X_2$ et $X_3$ ayant de préférence la même définition et n ayant la valeur 1, 2, 3 ou 4, d'un éther aliphatique ayant 2 à 20 atomes de carbone, d'un éther aromatique ayant 12 à 14 atomes de carbone, d'un éther aliphato-aromatique mixte ayant 7 à 24 atomes de carbone, d'une amine aliphatique tertiaire ayant 3 à 20 atomes de carbone ou d'une amine aromatique tertiaire ayant 8 à 24 atomes de carbone, les restes hydrocarbonés mentionnés pour $R^1$ pouvant en outre être substitués par des restes (alkoxy en $C_1$ à $C_4$)-carbonyle, CN, halogéno, ainsi que, dans le cas de restes aromatiques, par des radicaux alkyle inférieurs, comme activateurs pour percomposés.

3. Utilisation de composés de formule I, dans laquelle $R_2$, $R_3$ et m ont les définitions données dans la revendication 1 et $R^1$ est un reste $C_nH_{2n+1}$ avec n = 1-12, $-(CH_2)_m-$ avec m = 2-12,

$-[CH_2-\underset{R^8}{CH}-O]_p-CH_2-CH-$ avec $R^8$ = H, $CH_3$ et p = 1 à 9,

$-[CH_2-(CH_2)_q-\underset{R^9}{N}-]_r(CH_2)_q-CH_2-$ avec $R^9$ = $C_1$-$C_4$-Alkyle,

q = 1, 2 et r = 1-4,

(Voir dessin page 18)

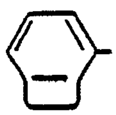 , 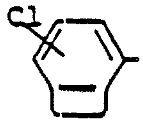 , 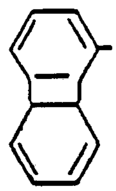 , 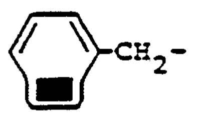 , 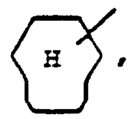 ,

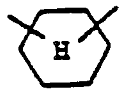 , 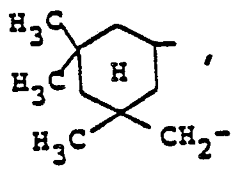 , 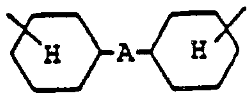

avec A = alkylène ayant 1 à 4 atomes de carbone,

$-O-$ ou 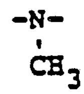 ,

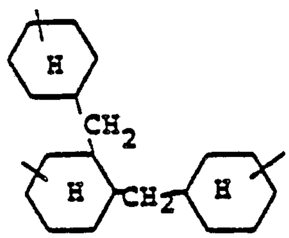 , 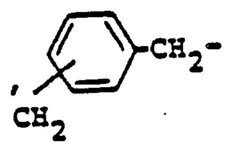 , 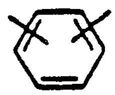 , 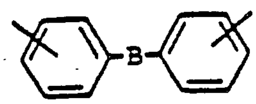

avec B = Alkylène ayant 1 à 4 atomes de carbone,

$-O-$ ou 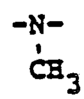 ,

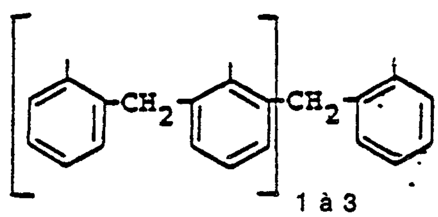
1 à 3

$C_4H_9—O—CH_2CH_2CH_2—$,

$—(CH_2)_3—O—(CH_2)_{2-4}—O—(CH_2)_3—$,

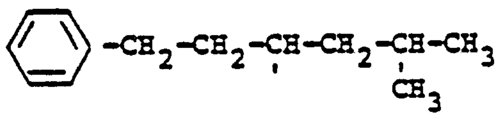

hydrogène,

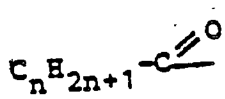

avec n = 1-3 ou

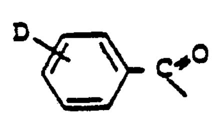

avec D = H, Cl, $CH_3$, $NO_2$ comme activateurs pour percomposés.

4. Utilisation de composés de formule I, dans laquelle $R_1$ et m ont les définitions indiquées dans la revendication 1, et dans laquelle $R^2$ et $R^3$ représentent un groupe (alkyle en $C_1$ à $C_8$)-carbonyle, benzoyle ou phényl-(alkyle en $C_1$ à $C_4$)-carbonyle, les restes acyle pouvant porter d'autres substituants tels que, par exemple, des substituants alkoxy en $C_1$ à $C_4$, halogéno, nitro et cyano, comme activateurs pour percomposés.

5. Utilisation de composés de formule I dans lesquels $R^1$ a la définition indiquée dans la revendication 1, m a la valeur 1 ou 2 et $R^2$, égal à $R^3$, représente un groupe acétyle, propionyle ou benzoyle ou bien m est égal à 1 et $R^1$, $R^2$ et $R^3$ sont égaux et représentent un groupe acétyle, propionyle ou benzoyle, comme activateurs pour percomposés.

6. Produits de blanchiment et/ou de lavage contenant des composés suivant les revendications 1 à 5 et un percomposé.

7. Produits de blanchiment et/ou de lavage, suivant la revendication 6, caractérisés en ce qu'ils contiennent des composés suivant les revendications 1 à 5 en une quantité telle qu'il y ait 0,2 à 6 restes acyle éliminables par atome d'oxygène actif du percomposé.

8. Produits de blanchiment et/ou de lavage, caractérisés en ce qu'ils contiennent 5 à 50 % en poids de percomposé et d'activateur suivant les revendications 1 à 5, 5 à 40 % en poids d'agents tensio-actifs, 10 à 80 % en poids de charges et 0-15 % en poids d'autres substances auxiliaires et additifs.